Europäisches Patentamt

(19) European Patent Office

Office européen des brevets

(11) Publication number: **0 352 866 B1**

# EUROPEAN PATENT SPECIFICATION

(12)

(45) Date of publication of patent specification:
20.02.91 Bulletin 91/08

(51) Int. Cl.⁵: **A61M 16/00**

(21) Application number: 89201970.4

(22) Date of filing: 26.07.89

(54) A mouth-to-mouth resuscitation mask.

(30) Priority: 27.07.88 NL 8801887

(43) Date of publication of application:
31.01.90 Bulletin 90/05

(45) Publication of the grant of the patent:
20.02.91 Bulletin 91/08

(84) Designated Contracting States:
ES GR

(56) References cited:
AU-B- 448 073
DE-A- 2 742 213
DE-U- 8 801 386
US-A- 3 802 428

(73) Proprietor: Daniels, Robertus Franciscus
Cornelia Maria
Nassaustraat 32
NL-5682 AD Best (NL)

(72) Inventor: Daniels, Robertus Franciscus
Cornelia Maria
Nassaustraat 32
NL-5682 AD Best (NL)

(74) Representative: Smulders, Theodorus A.H.J.,
Ir. et al
Vereenigde Octrooibureaux Nieuwe Parklaan
107
NL-2587 BP 's-Gravenhage (NL)

## Description

This invention relates to a mouth-to-mouth resuscitation mask comprising a foil of flexible synthetic plastics or like material, a portion of which is provided with a stiffened part whose centre contains an opening covered by an air-permeable material, the foil being made of non-transparent material.

It is an object of the present invention to improve such a mask disclosed in German Offenlegungsschrift 2742213.

To that effect, the mask is characterized in that the stiffened portion has the shape and size of a credit card and which mask is provided with a transparent eye window, so that when the victim of an accident has to be resuscitated, the eyes can be controled. Further the mask can easily be taken by someone as it has in folded condition the size of a credit card. Moreover, direct contact with blood is prevented.

By impregnating the air-permeable portion with a scent, the use of the mask for mouth-to-mouth resuscitation can be promoted in a simple manner.

In a further elaboration of the present invention, the foil may be provided circumferentially with an elastic edge, e.g. in the form of a separate piece of elastic, and the air permeable material is made in such a manner that air will flow more easily from the outside inwards than the other way round. Further only air but not fluids can pass the air permeable material. As a result, it is not only possible to put the mask and hence the air-permeable portion on the victim's head in a more or less fixed position, but it is moreover achieved that the mask can also serve to remedy hyperventilation problems : because the mask substantially seals over the face, the exhalation of too much air or nitrogen, producing the oppressed feeling in the event of hyperventilation, is prevented.

As in a lot of instances the victim shows blood and someone may "fear" to administer aid, the foil can have a surgical green or blood red colour.

In this simple manner, there is provided a life-saving aid that is easily carried along and can be used for both mouth-to-mouth resuscitation and to remedy hyperventilation symptoms.

For the sake of clearness it is remarked that from AU-B-448,073 in itself a mask for mouth-to-mouth resuscitation is known which has a special mouth piece, so that it is not foldable into a flat product that can easily be put away in for example a pocket-boot.

From DE-U-8,801,386 in itself is known a mask for mouth-to-mouth resuscitation comprising a foil of flexible synthetic plastics material, provided with a hole with an air-permeable material which material is electrostatic, so that bacteries will be hold back. Further resemblance is not there.

One embodiment of the mask according to the present invention will now be described, by way of example, with reference to the accompanying drawings, in which :

Fig. 1 is a front view of a mask according to the present invention worn on a head illustrated in stylized fashion ;

Fig. 2 is a cross-sectional view taken on the line II-II of Fig. 1 ;

Fig. 3 shows on a reduced scale the mask according to Figs. 1 and 2 in folded condition ; and

Fig. 4 is a cross-sectional view, on an enlarged scale, on the line IV-IV of Fig. 1.

As shown in the drawings, a mask is provided with a flexible foil 1 of synthetic plastics material wherein, at the location of the mouth of a head 2 shown in dash-dotted lines, there is provided a credit card-like, stiffened portion 3. The centre of stiffened portion 3 is provided with a hole 4 filled with a filler 7 covered on either side by a cover 5 of synthetic plastics material having apertures 8 therein. A suitable filter material is the material marketed by the company of Carl Freudenberg at Weinheim, BRD, under the name of Viledon Micro donfilter, type LRS 306.

Cover 5 can be made of synthetic plastics material, fine muslin, mull, gauze or the like : on the one hand a good air-permeability is required and on the other minimal contact between the victim and the donor. In order to render this aid "more agreable", said filter can be prepared with a scent, e.g. menthol, so that the donor is not troubled, or hardly so, by a possible unpleasant odour from the victim.

In this connection, it is observed that the foil can be coloured, so as to render any blood present on the victim inconspicuous, e.g. surgical green or blood red. In order that the victim's eyes may be observed, a transparent eye hole 9 is provided in the mask.

In order that the mask may also be used in the event the victim is suffering from hyperventilation, foil 1 may be provided with an elastic circumferential edge 6, so that the air or nitrogen exhaled by the victim remains underneath the mask, thereby preventing over-exhalation of air or nitrogen, producing the oppressed feeling or hyperventilation.

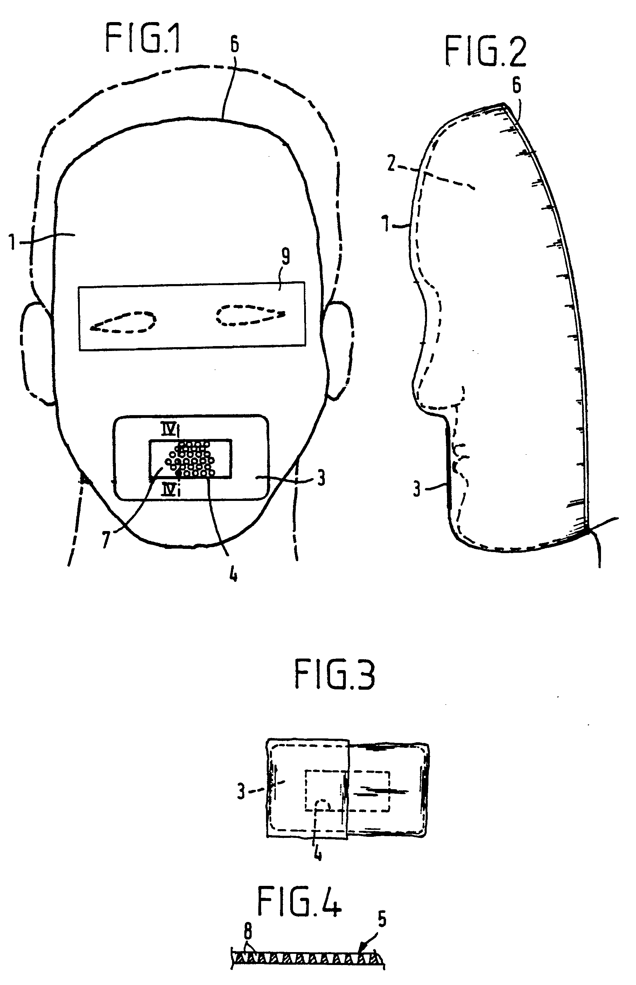

The illustration of the mask in folded condition in Fig. 3 appears to be self-explanatory. Possibly, the unit may be stored in a case carrying the bearer's personal data, such as name, address, blood group etc.

As noted above, the gauzy portion 5 should be made in such a manner that air or oxygen can be easily supplied therethrough to the victim, but on the other hand, this gauzy portion should be made in such a manner that the air cannot be easily discharged by the victim, in which case the contemplated effect in remedying hyperventilation would not occur. Preferably, the gauzy portion is made in such a manner that the air-permeability from the outside inwards is relatively high, but the air-permeability from the inside outwards is lower. This can be achieved e.g. by providing passages with a tapered configuration from the outside inwards : an example is shown in Fig. 4.

## Claims

1. A mouth-to-mouth resuscitation mask comprising a foil (1) of flexible synthetic plastics material, provided with a thickened stiffened portion (3) containing a hole (4) provided with an air-permeable material (5, 7, 8), the foil being made of non-transparent material, characterized in that the stiffened portion has the shape and size of a credit card and is provided with a transparent eye window (9).

2. A mask according to claim 1, characterized in that the foil is provided circumferentially with an elastic edge (6) and the air permeable material is made in such a manner that air flows more easily from the outside inwards than the other way round.

3. A mask as claimed in claim 2, characterized in that the air permeable material includes air passages which are tapered from the outside inwards.

4. A mask as claimed in claim 2 or 3, characterized in that only air but not fluids can pass the air permeable material.

5. A mask as claimed in any of the preceding claims, characterized in that it is packaged in a case on the outside of which the bearer's personal data can be written or otherwise displayed.

6. A mask as claimed in any one of the preceding claims, characterized in that the air-permeable material is impregnated with a scent, e.g. menthol.

7. A mask according to any one of the preceding claims, characterized in that the foil has a surgical green or blood red colour.

## Ansprüche

1. Mund-zu-Mund-Beatmungsmaske, versehen mit einer Folie (1) aus flexiblem Kunststoffmaterial, mit einem verdickten, versteiften Teil (3), der ein Loch (4) mit einem luftdurchlässigen Material (5, 7, 8) aufweist, welche Folie aus nicht-transparentem Material hergestellt ist, dadurch gekennzeichnet, daß der versteifte Teil die Form und Abmessung einer Kreditkarte aufweist und mit einem transparenten Augenfenster (9) versehen ist.

2. Maske nach Anspruch 1, dadurch gekennzeichnet, daß die Folie in Umfangsrichtung mit einem elastischen Rand (6) versehen ist und daß das luftdurchlässige Material so hergestellt ist, daß Luft leichter von außen nach innen strömt als umgekehrt.

3. Maske nach Anspruch 2, dadurch gekennzeichnet, daß das luftdurchlässige Material sich von außen nach innen verjüngende Luftdurchgänge aufweist.

4. Maske nach Anspruch 2 oder 3, dadurch gekennzeichnet, daß nur Luft, aber keine Flüssigkeiten das luftdurchlässige Material passieren kann.

5. Maske nach einem der vorhergehenden Ansprüche, dadurch gekennzeichnet, daß sie in einen Behälter verpackt ist, auf dessen Außenseite die Personalien des Inhabers geschrieben oder in anderer Weise gezeigt werden können.

6. Maske nach einem der vorhergehenden Ansprüche, dadurch gekennzeichnet, daß das luftdurchlässige Material mit einem Riechstoff, z.B. Menthol, imprägniert ist.

7. Maske nach einem der vorhergehenden Ansprüche, dadurch gekennzeichnet, daß die Folie eine chirurgische grüne oder blutrote Farbe aufweist.

## Revendications

1. Un masque pour réanimation par bouche à bouche comprenant une feuille de matière plastique flexible (1), munie d'une portion plus épaisse et rigidifiée (3), contenant un trou (4) muni d'un matériau perméable à l'air (5, 7, 8), la feuille étant en matière non transparente, caractérisée par le fait que la portion rigidifiée a la forme et la dimension d'une carte de crédit et est munie d'une fenêtre transparente pour les yeux (9).

2. Un masque similaire à la revendication 1, caractérisé par le fait que la circonférence de la feuille est pourvue d'une extrémité élastique (6) et que le matériau perméable à l'air est conçu de manière telle que l'air passe plus facilement de l'extérieur vers l'intérieur que l'inverse.

3. Un masque similaire à celui de la revendication 2, caractérisé par le fait que le matériau perméable à l'air comprend des passages d'air qui sont fuselés de l'extérieur vers l'intérieur.

4. Un masque similaire aux revendications 2 ou 3, caractérisé par le fait que seul l'air peut passer par le matériau perméable à l'air, et non des liquides.

5. Un masque similaire aux revendications précédentes, caractérisé par le fait qu'il est conditionné dans une boîte sur laquelle les données personnelles de son détenteur peuvent être écrites ou indiquées de toute autre manière.

6. Un masque similaire aux revendications précédentes, caractérisées par le fait que le matériau perméable à l'air est imprégné d'un odeur, de menthol, par exemple.

7. Un masque similaire aux revendications précédentes, caractérisé par le fait que la feuille est de couleur vert chirurgical ou rouge sang.

FIG.1

FIG.2

FIG.3

FIG.4